# EUROPÄISCHE PATENTSCHRIFT

(11) **EP 0 796 131 B1**
(45) Veröffentlichungstag und Bekanntmachung des Hinweises auf die Patenterteilung: **22.05.2002**
(21) Anmeldenummer: 95941624.9
(22) Anmeldetag: 23.11.1995
(51) Int. Cl.: A61N 5/06

(54) **VORRICHTUNG ZUM BESTRAHLEN VON KÖRPERGEWEBE MIT LASERLICHT**
APPARATUS FOR IRRADIATING BODY TISSUE WITH LASER LIGHT
DISPOSITIF DE TRAITEMENT D'UN TISSU CORPOREL PAR DE LA LUMIERE LASER

(30) Priorität: 09.12.1994 DE 4443964
(43) Veröffentlichungstag der Anmeldung: 24.09.1997
(73) Patentinhaber: Schwarzmaier, Hans-Joachim, 40229 Düsseldorf (DE)
(72) Erfinder: Schwarzmaier, Hans-Joachim, 40229 Düsseldorf (DE)
(74) Vertreter: Schaumburg, Thoenes & Thurn
(86) Internationale Anmeldenummer: EP9504629
(87) Internationale Veröffentlichungsnummer: WO9617655

(56) Entgegenhaltungen:
- EP-A- 0 214 712
- EP-A- 0 439 629
- EP-A- 0 495 443
- EP-A- 0 620 459
- EP-A- 0 629 380
- WO-A-93/15672
- WO-A-93/15677
- WO-A-93/20469
- DE-A- 4 137 983
- DE-A- 4 213 053
- US-A- 4 718 417

## Beschreibung

Die Erfindung betrifft eine Vorrichtung zum Bestrahlen von Körpergewebe mit Laserlicht hoher Intensität gemäß dem Oberbegriff des Anspruchs 1.

Seit 1983 wird der Laser zur interstiellen Erwärmung biologischen Gewebes benutzt. Zwischenzeitlich sind unterschiedliche Sonden und Applikatoren entwickelt worden, um das Laserlicht in humanes Gewebe einzustrahlen. Entsprechende Vorrichtungen sind in den deutschen Offenlegungsschriften DE-A-38 13 227, DE-A-41 37 983, DE-A-42 13 053 und DE-A-42 21 364 beschrieben.

Die bisher beschriebenen Systeme sind nach einem mehr oder weniger ähnlichen Prinzip konstruiert. Das Laserlicht ist über einen Lichtleiter in ein optisches Element eingekoppelt, das sich an der Spitze des Lichtleiters befindet. Hierbei handelt es sich in der Regel um Spiegel bzw. Prismen sowie spezielle Diffusoren, die eine gleichmäßige Abstrahlung des Laserlichtes in alle Raumrichtungen erlauben. Diese Systeme sind zur zusätzlichen Modifikation des Temperaturraumprofils im Gewebe teilweise mit einem Kühlkreislauf versehen, wie dies beispielsweise in der DE-A-42 21 364 beschrieben ist. Ein Hauptproblem aller interstiellen Erwärmungsverfahren ist jedoch die exakte Anpassung des Temperaturraumprofils an die vorgegebene Gewebeläsion. Dies soll am Beispiel der Prostatahyperplasie kurz skizziert werden.

Die Prostata gliedert sich anatomisch in einen rechten und einen linken Prostatalappen sowie einen Mittellappen. Die geometrische Ausdehnung ist zwar bei einer normalen Prostata - abgesehen von Größenunterschieden - ähnlich, die Ausprägungen der krankhaften Veränderungen sind jedoch von Patient zu Patient außerordentlich unterschiedlich. Es finden sich neben einer allgemeinen Vergrößerung der Prostata isolierte Veränderungen im rechten oder linken Prostatalappen sowie im Mittellappen. Außerdem können natürlich individuell sehr unterschiedliche Kombinationen dieser krankhaften Veränderungen auftreten. Darüber hinaus ergibt sich häufig, daß das Zielgewebe auf die Laserstrahlung unterschiedlich reagiert, obschon das Abstrahlprofil an die Läsion angepaßt wurde. Die Temperaturverteilung kann daher während der Bestrahlung unerwünscht zu hohe oder aber auch zu niedrige Temperaturen annehmen. Eine ähnliche Problematik ergibt sich bei unregelmäßig geformten Tumoren, wobei insbesondere die histologische Zusammensetzung innerhalb des Tumors (vitale Zellen, Nekrosen, Einblutungen, Gefäße) hier erschwerend hinzukommen kann. Eine Lasersonde, die diese Problematik löst, wurde bisher nicht beschrieben.

Der Erfindung liegt die Aufgabe zugrunde, eine Vorrichtung der eingangs genannten Art so zu konstruieren, daß sie bei einer Vielzahl spezieller pathologisch anatomischer Läsionsgeometrien einsetzbar ist und eine On-Line-Anpassung der Laserleistung an unterschiedliche Reaktionen innerhalb des bestrahlten Gewebes während des Bestrahlungsvorganges erlaubt.

Diese Aufgabe wird durch die Merkmale des Anspruchs 1 oder des Anspruchs 2 gelöst.

Ein erstes wesentliches Merkmal einer bevorzugten Ausführungsform der erfindungsgemäßen Lösung betrifft die Einkopplung des Laserlichtes in die Sonde sowie die Lichtleitung innerhalb derselben. Im Gegensatz zu den bekannten Systemen wird der Laserstrahl mittels einer Strahlteileranordnung in die erforderliche Anzahl von Teilstrahlen aufgespalten. Alternativ oder ergänzend dazu kann die Laserlichtquelle eine Mehrzahl einzelner Laser haben. Erst dann wird das Laserlicht in die Sonde eingekoppelt, die mehrere Lichtleiter umfaßt. Gemäß einem weiteren wesentlichen Merkmal ist zwischen der Strahlteileranordnung und der Sonde pro Teilstrahler eine Einrichtung zur Veränderung der Strahlintensität eingefügt, die beispielsweise mit Hilfe eines Schrittmotors stufenlos geregelt werden kann.

Die einzelnen Lichtleiter enden gewebeseitig, d.h. am distalen Ende der Sonde in einem Diffusorelement, das in an sich bekannter Weise aus einem Basismaterial besteht, welches für das verwendete Laserlicht hochtransparent ist. In dieses Material wird eine definierte Anzahl von Streupartikeln eingebracht. Als ein wesentliches Merkmal sind die einzelnen Diffusoren erfindungsgemäß jedoch mit einem hochreflektierenden Material gekapselt, wobei der so gebildete Reflektor lediglich im Bereich der gewünschten Abstrahlungsrichtung eine Öffnung aufweist. Hier ist insbesondere darauf hinzuweisen, daß eine Umlenkung des Strahles mit Hilfe eines Prismas oder Spiegels im Lichtaustrittsbereich nicht für eine wirksame Bestrahlung ausreicht. Der Strahl muß vielmehr so homogen wie möglich über den gesamten Austrittsbereich verteilt sein, da die ansonsten auftretenden hohen Leistungsdichten zu einer Überhitzung des sondennahen Gewebes führen. Andererseits ist jedoch ein Diffusor - auch bei asymmetrischer Verteilung der Streupartikel, wie z.B. in der DE-A-42 21 364 beschrieben - ohne reflektierende Ummantelung unzureichend geeignet, den Lichtaustritt in eine unerwünschte Richtung vollständig zu unterbinden. Bei der erfindungsgemäßen Lösung sorgt der den Diffusor umgebende Reflektor also dafür, daB Licht aus dem jeweiligen Lichtleiter nur in die gewünschte Richtung abgestrahlt wird. Damit können Richtung und Intensität des abgestrahlten Laserlichtes entsprechend der jeweiligen Geometrie des zu bestrahlenden Gewebes gewählt werden. Die Anzahl der Abstrahlelemente wird entsprechend dem jeweiligen Anwendungsfall gewählt.

Diese Maßnahmen zur Modifizierung der initialen Photonenverteilung im Gewebe sind jedoch für sich genommen zur Problemlösung ungeeignet, da durch Temperaturleitung im Gewebe trotz asymmetrischer Einstrahlung wieder eine symmetrische Läsion entsteht. Erst die Kühlung des sondennahen Gewebes über die Sonde gewährleistet die asymmetrische Läsion, da hierdurch der durch Lichtabsorption erzeugte Temperaturgradient im Gewebe erhalten bleibt und ein Temperaturausgleich zwischen bestrahlten und nicht bestrahlten Gewebebereichen verhindert wird. Darüber hinaus dient der Kühlkreislauf zur zusätzlichen Modifikation des Temperaturraumprofils.

Die On-Line-Regelung der Laser-Gewebe-Wechselwirkung erfordert eine adäquate Temperaturüberwachung. Je nach Ausführung der Vorrichtung ist die Sonde daher zusätzlich mit mindestens einem Temperatursensor, beispielsweise einem Thermoelement versehen.

Punktuelle Temperaturmessungen sind bei asymmetrischen Temperaturraumprofilen in der Regel unzureichend. Dies gilt insbesondere für Indikationen, bei denen aufgrund von Gewebeinhomogenität unterschiedliche Temperaturverläufe zu erwarten sind. Hier ist für eine aussagekräftige Temperaturmessung eine dreidimensionale Temperaturkontrolle erforderlich. Eine praktikable Lösung stellt die Überwachung des Bestrahlungsvorganges mit Hilfe der Kernspintomografie dar. Die derzeit verwendeten Ganzkörperspulen sind jedoch hinsichtlich ihrer Auflösung von Details, beispielsweise der Harnröhrenwand, begrenzt.

Gemäß einer bevorzugten Ausführungsform der Erfindung umfaßt daher die Sonde eine zusätzliche Antenne, um die zur Diagnostik mittels üblicher Kernspintomografen erforderliche Hochfrequenzstrahlung direkt lokal zu applizieren. Darüber hinaus kann die Antenne auch als Empfangsantenne geschaltet oder eine weitere Empfangsantenne vorgesehen sein, um die von dem Kernspintomografen ausgesandte HF-Strahlung aufzunehmen. Diese Antenne kann auch zur Applikation therapeutischer Strahlungsdosen benutzt werden.

Vorteilhafte Ausgestaltungen der Erfindung sind in den übrigen Unteransprüchen angegeben.

Weitere Merkmale und Vorteile der Erfindung ergeben sich aus der folgenden Beschreibung, die in Verbindung mit den beigefügten Zeichnungen die Erfindung anhand von Ausführungsbeispielen erläutert. Es zeigen:
- Figur 1: eine schematische Darstellung des mit dem proximalen Ende der Sonde verbundenen Teiles der Vorrichtung mit einer Laserlichtquelle, einer Strahlteileranordnung und einer Wärmetauschereinrichtung,
- Figur 2: einen schematischen, die Achse enthaltenden Schnitt durch den distalen Endbereich der Sonde,
- Figuren 3 bis 6: jeweils einen schematischen Querschnitt durch die Sonde entlang der Linie III-III in Figur 2, wobei das äußere Hüllrohr der Sonde nicht dargestellt ist,
- Figur 7: eine der Figur 2 entsprechende Schnittdarstellung durch eine Sonde gemäß einer zweiten Ausführungsform der Erfindung,
- Figur 8: einen den Figuren 3 bis 6 entsprechenden Schnitt durch die zweite Ausführungsform der Sonde entlang Linie VIII-VIII in Figur 7,
- Figur 9: eine schematische Darstellung des praktischen Einsatzes der Sonde bei der Bestrahlung der Prostata, und
- Figur 10: einen Schnitt entlang Linie X-X in Figur 7.

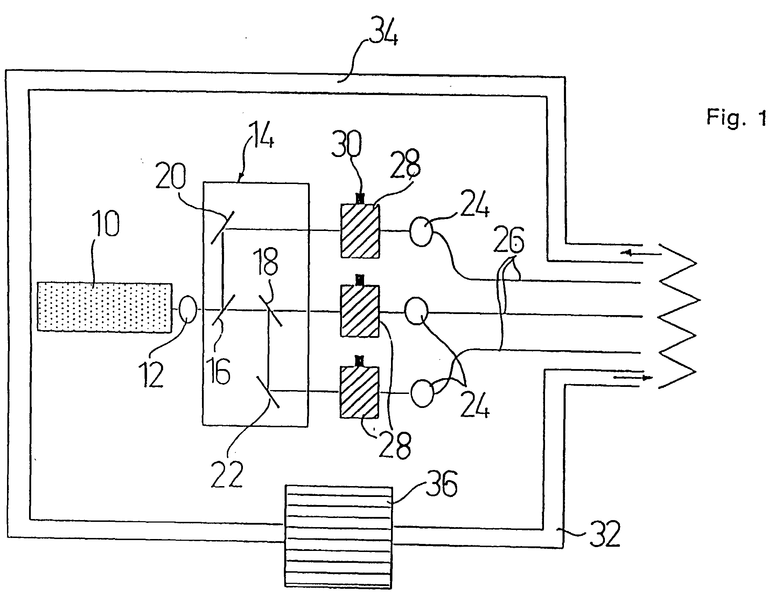

Figur 1 zeigt einen Laser 10, dessen Ausgangsstrahl mittels einer Optik 12 in eine Strahlteileranordnung 14 eingekoppelt wird. Die Strahlteileranordnung umfaßt hier beispielsweise zwei hintereinander angeordnete Strahlteiler 16, 18 und zwei Spiegel 20, 22, die den eingekoppelten Strahl des Lasers 10 in der dargestellten Weise in drei Teilstrahlen aufteilen. Die Anzahl der Strahlteiler muß natürlich je nach der gewünschten Anzahl der Teilstrahlen variiert werden. Die Teilstrahlen werden jeweils über eine Optik 24 in je einen Lichtleiter 26 eingekoppelt. Zwischen dem Ausgang der Strahlteileranordnung 14 und der jeweiligen Optik 24 ist in jedem Teilstrahl eine Einrichtung zur Veränderung der Intensität des Teilstrahles angeordnet, beispielsweise ein variables Filter 28. Die Abschwächung des Strahles kann alternativ auch mit rotierenden Spiegelprismen oder Glasplatten erfolgen. Das variable Filter kann beispielsweise mit Hilfe eines Schrittmotors 30 stufenlos geregelt werden.

Die in Figur 1 dargestellte Vorrichtung umfaßt ferner einen Kühlkreislauf zum Kühlen der Sonde mit einem Zulauf 32, einem Ablauf 34 und einer Pumpe/Wärmetauschereinheit 36 zum Umpumpen und Temperieren des Kühlmittels.

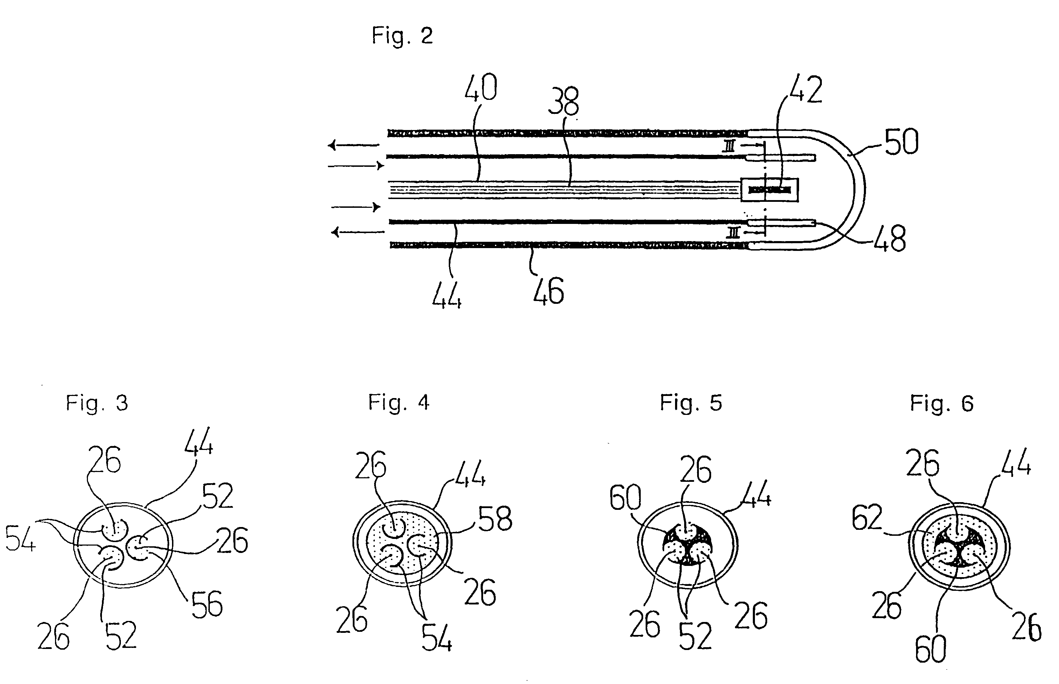

An die in der Figur 1 schematisch dargestellte Vorrichtung schließt sich die eigentliche Sonde an, deren distaler Endabschnitt in Figur 2 dargestellt ist. Im Zentrum der in das Gewebe einzuführenden Sonde befindet sich ein aus den Lichtleitern 26 bestehende Faserbündel 38, das zur besseren mechanischen Stabilität zweckmäßigerweise von einer zusätzlichen Umhüllung 40 (z.B. Schlauch, Rohr) umgeben ist. Die Lichtleiter oder Lichtleitfasern 26 enden distal in einem Abstrahlkopf 42, das anhand der Figuren 3 bis 6 noch näher erläutert wird. Das Faserbündel 38 ist mit radialem Abstand von einem inneren ersten Hüllschlauch oder Hüllrohr 44 umgeben, das an seinem distalen Ende offen ist und den Zulauf 32 für das Kühlmittel bildet. Dieses innere Hüllrohr ist seinerseits mit radialem Abstand von einem äußeren zweiten Hüllrohr 46 umgeben, das an seinem distalen Ende geschlossen ist und den Abfluß oder Rücklauf für das Kühlmittel bildet. Der den Abstrahlkopf 42 umgebende Endabschnitt 48 des inneren Hüllrohres und der entsprechende kappenförmig geschlossene Endabschnitt 50 des äußeren Hüllrohrs 46 sind aus einem für die jeweils verwendete Laserstrahlung gut transparenten Material hergestellt. Das für die transparente Verschlußkappe 50 verwendete Material muß darüber hinaus gut wärmeleitend sein.

Der Abstrahlkopf 42 wird nun anhand der Figuren 3 bis 6 näher erläutert.

Bei der Ausführungsform gemäß Figur 3 endet jeder Lichtleiter 26 in einem Diffusor 52. Als Material für den Diffusor wird eine Kombination aus einem hochtransparenten Basismaterial (z.B. Methylmetacrylat oder ähnliche Stoffe, Glas oder glasähnliche Stoffe) und eingelagerten Streuzentren (z.B. Bariumsulfat, Magnesiumoxid oder ähnliche streuende Stoffe, Luftblasen bzw. Schaumglas) benutzt. Der Diffusor ist jeweils von einer hochreflektierenden Hülle 54 teilweise eingeschlossen. Der so gekapselte Diffusor weist nur in dem gewünschten Abstrahlbereich eine Öffnung 56 auf. Als Kapselmaterial kommt jedes für die verwendete Wellenlänge hochreflektierende Material in Frage (beispielsweise Gold, Aluminium und dergleichen).

Bei der in Figur 4 dargestellten Ausführungsform sind die drei teilweise gekapselten Diffusoren insgesamt in einen Diffusor 58 eingebettet. Gegebenenfalls könnten bei dieser Lösung die inneren Diffusoren 52 entfallen.

Bei der in der Figur 5 dargestellten Ausführungsform werden die die einzelnen Diffusoren 52 umgebenden Reflektoren von einem einzigen zylindrischen Element aus einem hochreflektierenden Material umgeben, das die Lichtleiterendabschnitte und die Diffusoren 52 aufnimmt und im Abstrahlbereich wieder entsprechenden Öffnungen 56 hat. Auch dieses Element 60 kann wie bei der Ausführungsform gemäß Figur 4 in einen äußeren Diffusor 62 eingebettet sein, wie dies Figur 6 zeigt. Dies erlaubt eine weitere Modifizierung des mit der Sonde zu erreichenden Temperaturraumprofils.

Die jeweiligen inneren Diffusoren 52 und äußeren Diffusoren 58, 62 können hinsichtlich ihrer Streueigenschaften entweder durch Änderung der Verteilung der Streupartikel im Basismaterial oder aber durch eine Veränderung der Form des Diffusors modifiziert werden. Eine Änderung der Form des Innendiffusors erfordert natürlich auch eine Änderung der Form der hochreflektierenden Hülle bzw. des Reflektors.

Wie man erkennt, wird durch die erfindungsgemäß vorgesehenen, die Enden der Lichtleiter 26 teilweise einschließenden Reflektoren 54, 60 das gestreute Licht nur in bestimmte Richtungen abgegeben. In Verbindung mit einer Steuerung der Intensität des eingekoppelten Lichtes über die Filterelemente 28 kann somit Intensität und Richtung des abgestrahlten Laserlichtes gesteuert und damit das Abstrahlprofil an die Läsion des bestrahlten Gewebes angepaßt werden.

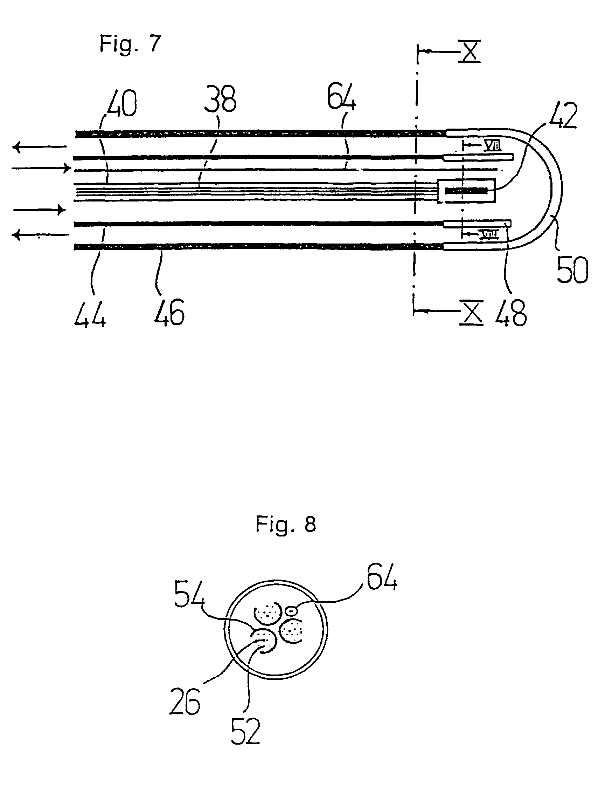

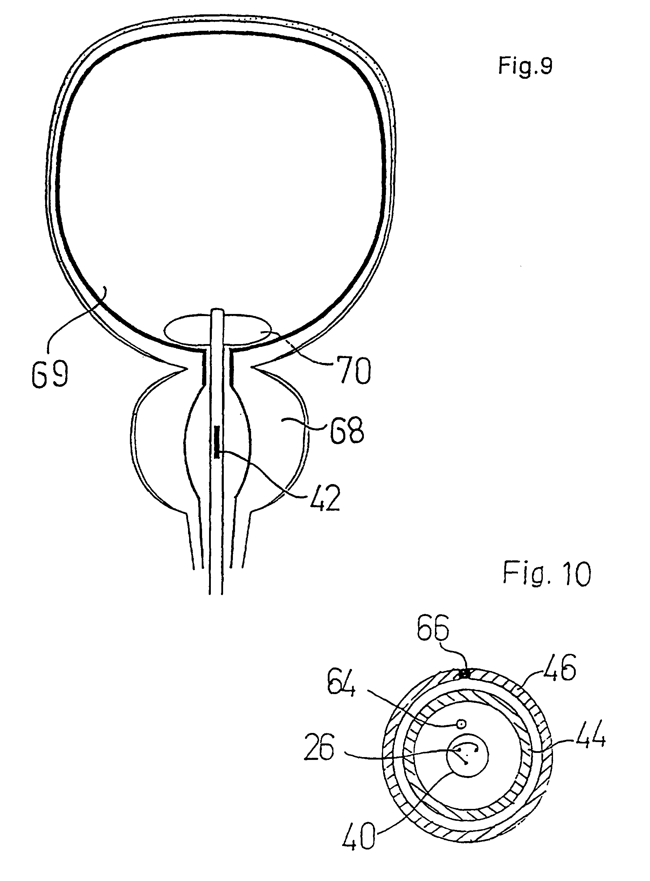

Das umlaufende Kühlmittel erlaubt eine Modifikation des mittels der abgestrahlten Energie erreichten Temperaturraumprofils. Die Überwachung des Temperaturraumprofils kann durch Temperaturmessung mittels einer (Temperaturfühler 66 in Figur 10) oder mehrerer Temperatursonden, vorzugsweise aber mit einem Kernspintomografen erfolgen. Zu diesem Zwekke ist bei der Ausführungsform gemäß Figur 7 innerhalb des inneren Hüllrohres 44 der Sonde eine Antenne 64 vorgesehen, die eine lokale Einstrahlung der Hochfrequenzenergie bzw. einen Empfang der entsprechenden Signale für die kernspintomografische Überwachung ermöglicht. Die Antenne kann natürlich auch zur simultanen Einstrahlung therapeutischer Strahlungsdosen von Hochfrequenzenergie geeigneter Wellenlänge (z.B. 2,45 GHz) genutzt werden, was eine zusätzliche Modifikation des Temperaturraumprofils erlaubt. Das Diffusorelement ist dabei wie bei der Ausführungsform gemäß Figur 3 ausgebildet.

Der Kühlkreislauf kann in Abwandlung der in den Figuren 2 und 7 dargestellten Ausführungsformen unterschiedlich ausgebildet werden.

Die Sonde kann zur Anpassung an die anatomischen Gegebenheiten ihres jeweiligen Einsatzgebietes unterschiedlich ausgebildet sein. So wird zum Einsatz der Sonde bei der Prostata die Abstrahleinheit nicht im Bereich der Spitze liegen, wie dies bei den Ausführungsformen gemäß den Figuren 2 und 7 der Fall ist. Gemäß Figur 9 wird der Abstrahlkopf 42 so positioniert, daß er innerhalb der Prostata 69 liegt, die Spitze der Sonde aber bis in die Blase 68 vorgeschoben werden kann. Bei einer solchen Ausführungsform sind gegebenenfalls zusätzliche Positionierhilfen erforderlich, beispielsweise ein Ballon 70. Dieser könnte entweder in der Harnblase oder aber in der Harnröhre selbst positioniert werden. Bei einer konstruktiven Variante könnte dieser Ballon auch die äußere Hülle der Sonde ersetzen. Der Abfluß des Kühlmittels würde in diesem Fall zwischen dem inneren Hüllröhrchen und dem positionierenden Ballon erfolgen, wobei dieser dann wiederum für das verwendete Laserlicht gut transparent und ausreichend wärmeleitend sein müßte.

## Patentansprüche

1. Vorrichtung zum Bestrahlen von Körpergewebe mit Laserlicht hoher Intensität, umfassend
eine Sonde mit einer Mehrzahl von Lichtleitern (26), an deren jeweiligem in das Körpergewebe einführbaren Lichtaustrittsabschnitt ein Diffusorelement (52) vorgesehen ist, das aus einem für das Laserlicht hochtransparenten Basismaterial und in dieses eingelagerten, die Lichtstrahlen ablenkenden Streupartikeln besteht, wobei jeder Lichtleiter (26) im Bereich seines Lichtaustrittsabschnittes von einem Reflektor (54) und/oder Absorber umgeben ist, der im Bereich der gewünschten Abstrahlfläche mindestens eine Lichtaustrittsöffnung (56) hat, sowie mit einem die Lichtleiter (26) mit Abstand umgebenden, an seinem distalen Ende offenen ersten Hüllrohr (44), das mindestens im Bereich des Diffusorelementes (52) aus einem transparenten und gut wärmeleitenden Material besteht, und einem das erste Hüllrohr mit Abstand umgebenden zweiten Hüllrohr (46), das im Abstrahlbereich der Sonde durch eine transparente, gut wärmeleitende Kappe (50) verschlossen ist, wobei der Innenraum des ersten Hüllrohres (44) an einen Kühlmittelzufluß (32) und der Ringraum zwischen den beiden Hüllrohren (44, 46) an einen Kühlmittelabfluß (34) angeschlossen ist,
eine mit den Lichtleitern (26) koppelbare Laserlichtquelle (10) und
eine zum Aufspalten des Strahles der Laserlichtquelle (10) in Teilstrahlen bestimmte Strahlteileranordnung (14), die zwischen der Laserlichtquelle (10) und den Lichteintrittsenden der Lichtleiter (26) so positioniert ist, daß in die Lichtleiter (26) jeweils ein Teilstrahl eingekoppelt wird, wobei im Wege jedes Teilstrahles eine Einrichtung (28, 30) zur Veränderung der Strahlintensität angeordnet ist.

2. Vorrichtung zum Bestrahlen von Körpergewebe mit Laserlicht hoher Intensität, umfassend eine
Sonde mit einer Mehrzahl von Lichtleitern (26), an deren jeweiligem in das Körpergewebe einführbaren Lichtaustrittsabschnitt ein Diffusorelement (52) vorgesehen ist, das aus einem für das Laserlicht hochtransparenten Basismaterial und in dieses eingelagerten, die Lichtstrahlen ablenkenden Streupartikeln besteht, wobei jeder Lichtleiter (26) im Bereich seines Lichtaustrittsabschnittes von einem Reflektor (54) und/oder Absorber umgeben ist, der im Bereich der gewünschten Abstrahlfläche mindestens eine Lichtaustrittsöffnung (56) hat, sowie mit einem die Lichtleiter (26) mit Abstand umgebenden, an seinem distalen Ende offenen ersten Hüllrohr (44), das mindestens im Bereich des Diffusorelementes (52) aus einem transparenten und gut wärmeleitenden Material besteht, und einem das erste Hüllrohr mit Abstand umgebenden zweiten Hüllrohr (46), das im Abstrahlbereich der Sonde durch eine transparente, gut wärmeleitende Kappe (50) verschlossen ist, wobei der Innenraum des ersten Hüllrohres (44) an einen Kühlmittelzufluß (32) und der Ringraum zwischen den beiden Hüllrohren (44, 46) an einen Kühlmittelabfluß (34) angeschlossen ist,
und eine Laserlichtquelle mit einer Mehrzahl einzelner Laser zum Erzeugen von Teilstrahlen, die jeweils in einen Lichtleiter (26) einkoppelbar sind, wobei im Wege jedes Teilstrahles eine Einrichtung (28, 30) zur Veränderung der Strahlintensität angeordnet ist.

3. Vorrichtung nach Anspruch 1, **dadurch gekennzeichnet, daß** die Strahlteileranordnung (14) bei mehr als zwei Lichtleitern (26) eine Mehrzahl aufeinanderfolgender Strahlteilerelemente (16, 18) umfaßt.

4. Vorrichtung nach einem der Ansprüche 1 bis 3, **dadurch gekennzeichnet, daß** die Einrichtung (28, 30) zur Veränderung der Strahlintensität ein Absorptionselement (28) umfaßt.

5. Vorrichtung nach Anspruch 4, **dadurch gekennzeichnet, daß** das Absorptionselement (28) ein Graufilter ist.

6. Vorrichtung nach einem der Ansprüche 1 bis 3, **dadurch gekennzeichnet, daß** die Einrichtung zur Veränderung der Strahlintensität eine Anordnung zum Reflektieren des jeweiligen Teilstrahles umfaßt.

7. Vorrichtung nach einem der Ansprüche 1 bis 6, **dadurch gekennzeichnet, daß** der Lichtaustrittsabschnitt jedes Lichtleiters (26) in ein Diffusorelement (52) eingebettet ist, das von dem Reflektor (54) umschlossen ist.

8. Vorrichtung nach Anspruch 7, **dadurch gekennzeichnet, daß** die Reflektoren (54) gemeinsam in einen äußeren Diffusor (58) eingebettet sind.

9. Vorrichtung nach einem der Ansprüche 1 bis 6, **dadurch gekennzeichnet, daß** die die Lichtaustrittsabschnitte der Lichtleiter (26) umgebenden Reflektoren (54) in einen äußeren Diffusor (58) eingebettet sind.

10. Vorrichtung nach einem der Ansprüche 1 bis 9, **dadurch gekennzeichnet, daß** die Reflektoren die teilzylindrischen Wände von Bohrungen sind, die in einem Reflektorkörper (60) ausgebildet sind.

11. Vorrichtung nach einem der Ansprüche 1 bis 10, **dadurch gekennzeichnet, daß** das Basismaterial für den Diffusor (52, 58, 62) aus der Methylmetacrylate, Glas und flexible transparente Kunststoffe umfassenden Materialgruppe ausgewählt ist und daß das Material für die Streupartikel aus der Bariumsulfat, Magnesiumoxid und Aluminium umfassenden Materialgruppe ausgewählt ist.

12. Vorrichtung nach einem der Ansprüche 1 bis 11, **dadurch gekennzeichnet, daß** die Streupartikel in den Diffusoren aus Glas mit von dem Basismaterial verschiedenem Brechungsindex oder mit Glaseinschlüssen bestehen.

13. Vorrichtung nach einem der Ansprüche 1 bis 12, **dadurch gekennzeichnet, daß** die Lichtleiter (26) als Flüssiglichtleiter ausgeführt und an den Kühlmittelzulauf angeschlossen sind.

14. Vorrichtung nach einem der Ansprüche 1 bis 13, **dadurch gekennzeichnet, daß** die Sonde mit einer Positionierhilfe in Form mindestens eines Ballons (70) versehen ist.

15. Vorrichtung nach Anspruch 14, **dadurch gekennzeichnet, daß** die Positionierhilfe sich im Bereich der Austrittsöffnung des Laserlichtes befindet und für das verwendete Laserlicht transparent und gut wärmeleitend ist.

16. Vorrichtung nach Anspruch 14, **dadurch gekennzeichnet, daß** die Positionierhilfe (70) außerhalb des Abstrahlbereiches der Sonde angeordnet ist.

17. Vorrichtung nach einem der Ansprüche 1 bis 16, **dadurch gekennzeichnet, daß** die Sondenaußenhülle (46) starr ist.

18. Vorrichtung nach einem der Ansprüche 1 bis 16, **dadurch gekennzeichnet, daß** die Sondenaußenhülle (46) flexibel ist.

19. Vorrichtung nach einem der Ansprüche 1 bis 18, **dadurch gekennzeichnet, daß** die Sonde mindestens einen Temperatursensor hat.

20. Vorrichtung nach einem der Ansprüche 1 bis 19, **dadurch gekennzeichnet, daß** die Sonde aus kernspinkompatiblen Materialien hergestellt ist.

21. Vorrichtung nach einem der Ansprüche 1 bis 20, **dadurch gekennzeichnet, daß** die Sonde in Form eines Katheters ausgeführt ist, dessen distales Ende mit einer zum Einstechen in festes Gewebe geeigneten Spitze versehen ist.

22. Vorrichtung nach einem der Ansprüche 1 bis 21, **dadurch gekennzeichnet, daß** die Sonde mindestens eine Antenne zum Einstrahlen und/oder Empfangen von Hochfrequenzenergie hat.

## Claims

1. Device for irradiating body tissues with laser light of high intensity, said device comprising
a probe with a plurality of waveguides (26), at the respective light exit section of which, being insertable into body tissue, is provided one diffuser element (52) each, which diffuser element consists of a base material that is highly transparent for the laser light and of scattering particles dispersed in said base material, which particles deflect the light rays, each waveguide (26) being surrounded by a reflector (54) and/or absorber in the area of its light exit section, said reflector (54) and/or absorber having at least one light exit opening (56) in the area of the desired radition surface, and with a first cover tube (44) surrounding the waveguides (26) at a distance and being open at its distal end, said cover tube being made of a transparent and good heat conducting material at least in the area of the diffuser element (52), as well as a second cover tube (46) surrounding the first cover tube at a distance, said second cover tube being closed off in the irradiation area of the probe by a transparent cap (50) which conducts heat well, the interior of the first cover tube (44) being connected to a coolant supply (32) and the annulus between the two cover tubes (44, 46) being connected to a coolant outflow (34),
a laser light source (10) which is couplable with the waveguides (26), and
a beam-splitting device (14) for splitting the beam of the laser light source (10) into component beams, said beam splitting device being positioned between the laser light source (10) and the light entry ends of the waveguides (26) such that one component beam each is launched in the waveguides (26), an installation (28, 30) being arranged in the path of each component beam for changing the beam intensity.

2. Device for irradiating body tissues with laser light of high intensity, said device comprising
a probe with a plurality of waveguides (26), at the respective light exit section of which, being insertable into body tissue, is provided one diffuser element (52) each, which diffuser element consists of a base material that is highly transparent for the laser light and of scattering particles dispersed in said base material, which particles deflect the light rays, each waveguide (26) being surrounded by a reflector (54) and/or absorber in the area of its light exit section, said reflector (54) and/or absorber having at least one light exit opening (56) in the area of the desired radition surface, and with a first cover tube (44) surrounding the waveguides (26) at a distance and being open at its distal end, said first cover tube being made of a transparent and good heat conducting material at least in the area of the diffuser element (52), as well as a second cover tube (46) surrounding the first cover tube at a distance, said second cover tube being closed off in the irradiation area of the probe by a transparent cap (50) which conducts heat well, the interior of the first cover tube (44) being connected to a coolant supply (32) and the annulus between the two cover tubes (44, 46) being connected to a coolant outflow (34),
and a laser light source having a plurality of individual lasers for generating component beams, each of which can be launched in a waveguide (26), an installation (28, 30) being arranged in the path of each component beam for changing the beam intensity.

3. Device according to claim 1, **characterized in that** the beam-splitting device (14) comprises a plurality of successive beam-splitting elements (16, 18) given more than two waveguides (26).

4. Device according to one of the claims 1 to 3, **characterized in that** the device (28, 30) comprises an absorption element (28) for changing the beam intensity.

5. Device according to claim 4, **characterized in that** the absorption element (28) is a neutral filter.

6. Device according to one of the claims 1 to 3, **characterized in that** the device for changing the beam intensity comprises an arrangement for reflecting the respective component beam.

7. Device according to one of the claims 1 to 6, **characterized in that** the light exit section of each waveguide (26) is embedded in a diffuser element (52) which is enclosed by the reflector (54).

8. Device according to claim 7, **characterized in that** the reflectors (54) are jointly embedded in an external diffuser (58).

9. Device according to one of the claims 1 to 6, **characterized in that** the reflectors (54) surrounding the light exit sections of the waveguides (26) are embedded in an external diffusor (58).

10. Device according to one of the claims 1 to 9, **characterized in that** the reflectors are the partial cylindrical walls of bores formed in a reflector body (60).

11. Device according to one of the claims 1 to 10, **characterized in that** the base material for the diffuser (52, 58, 62) is selected from the materials group comprising methyl methacrylate, glass and flexible transparent plastics and **in that** the material for the scattering particles is seleted from the materials group comprising barium sulfate, magnesium oxide and aluminium.

12. Device according to one of the claims 1 to 11, **characterized in that** the scattering particles in the diffusers consist of glass with a refraction index different from the base material or with glass inclusions.

13. Device according to one of the claims 1 to 12, **characterized in that** the waveguides (26) are constructed as liquid waveguides and are connected to the coolant inflow.

14. Device according to one of the claims 1 to 13, **characterized in that** the probe is provided with a positioning aid in the form of at least one balloon (70).

15. Device according to claim 14, **characterized in that** the positioning aid is situated in the area of the exit opening of the laser light and is transparent and a good heat conductor for the laser light used.

16. Device according to claim 14, **characterized in that** the positioning aid (70) is arranged outside the irradiation area of the probe.

17. Device according to one of the claims 1 to 16, **characterized in that** the outer probe casing (46) is rigid.

18. Device according to one of the claims 1 to 16, **characterized in that** the outer probe casing (46) is flexible.

19. Device according to one of the claims 1 to 18, **characterized in that** the probe has at least one temperature sensor.

20. Device according to one of the claims 1 to 19, **characterized in that** the probe is manufactured of nuclear spin-compatible materials.

21. Device according to one of the claims 1 to 20, **characterized in that** the probe is constructed in the form of a catheter, the distal end of which is provided with a tip suitable for insertion into solid tissue.

22. Device according to one of the claims 1 to 21, **characterized in that** the probe has at least one antenna for irradiation and/or reception of high frequency energy.

## Revendications

1. Appareil pour exposer un tissu. d'un corps à une lumière laser de forte intensité, comprenant :
* une sonde comportant
- une pluralité de guides de lumière (26) pour chacun desquels il est prévu, au niveau de la portion de sortie de la lumière, insérable dans le tissu du corps, un élément diffuseur (52) qui est constitué d'une matière de base très transparente à la lumière laser et de particules de dispersion déviant les rayons incorporées dans ladite matière de base, chaque guide de lumière (26) étant entouré, au niveau de sa portion de sortie de la lumière, d'un réflecteur (54) et/ou d'un absorbeur, qui comporte au niveau de la face de rayonnement souhaitée au moins une ouverture de sortie de lumière (56),
- ainsi qu'une première gaine (44), ouverte à son extrémité distale et entourant à distance les guides de lumière (26) , qui est constituée, au moins au niveau de l'élément diffuseur (52), d'une matière transparente ayant une bonne conductibilité thermique, et
- une seconde gaine (46), entourant à distance la première gaine, qui est fermée dans la zone de rayonnement de la sonde par un capuchon (50) transparent ayant une bonne conductibilité thermique, l'espace intérieur de la première gaine (44) étant raccordé à une amenée d'agent de refroidissement (32), et l'espace annulaire entre les deux gaines (44, 46) étant raccordé à une sortie d'agent de refroidissement (34),
* une source de lumière laser (10) pouvant être couplée aux guides de lumière (26), et
* un dispositif séparateur de faisceau (14), destiné à séparer le faisceau émis par la source de lumière laser (10) en faisceaux secondaires, qui est positionné entre la source de lumière laser (10) et les extrémités d'entrée de la lumière des guides de lumière (26), de telle sorte qu'un faisceau secondaire est couplé à chacun des guides de lumière (26), un dispositif variateur d'intensité de faisceau (28, 30) étant agencé sur le trajet de chaque faisceau secondaire.

2. Appareil pour exposer un tissu d'un corps à une lumière laser de forte intensité, comportant :
* une sonde comportant
- une pluralité de guides de lumière (26) pour chacun desquels il est prévu, au niveau de la portion de sortie de la lumière, insérable dans le tissu du corps, un élément diffuseur (52) qui est constitué d'une matière de base très transparente à la lumière laser et de particules de dispersion déviant les rayons incorporées dans ladite matière de base, chaque guide de lumière (26) étant entouré, au niveau de sa portion de sortie de la lumière, d'un réflecteur (54) et/ou d'un absorbeur, qui comporte au niveau de la face de rayonnement souhaitée au moins une ouverture de sortie de lumière (56),
- ainsi qu'une première gaine (44), ouverte à son extrémité distale et entourant à distance les guides de lumière (26), qui est constituée, au moins au niveau de l'élément diffuseur (52), d'une matière transparente ayant une bonne conductibilité thermique, et
- une seconde gaine (46), entourant à distance la première gaine, qui est fermée dans la zone de rayonnement de la sonde par un capuchon (50) transparent ayant une bonne conductibilité thermique, l'espace intérieur de la première gaine (44) étant raccordés à une amenée d'agent de refroidissement (32), et l'espace annulaire entre les deux gaines (44, 46) étant raccordé à une sortie d'agent de refroidissement (34),
* et une source de lumière laser comportant une pluralité de lasers individuels pour générer des faisceaux secondaires qui sont chacun couplés à un guide de lumière (26), un dispositif variateur d'intensité de faisceau (28, 30) étant agencé sur le trajet de chaque faisceau secondaire.

3. Appareil selon la revendication 1, **caractérisé en ce que** le dispositif séparateur de faisceau (14) comporte une pluralité d'éléments séparateurs de faisceau (16, 18) successifs lorsqu'il y a plus de deux guides de lumière (26).

4. Appareil selon l'une des revendications 1 à 3, **caractérisé en ce que** le dispositif variateur d'intensité de faisceau (28, 30) comporte un élément d'absorption (28).

5. Appareil selon la revendication 4, **caractérisé en ce que** l'élément d'absorption (28) est un filtre gris.

6. Appareil selon l'une des revendications 1 à 3, **caractérisé en ce que** le dispositif variateur d'intensité de faisceau comporte un dispositif destiné à réfléchir le faisceau secondaire correspondant.

7. Appareil selon l'une des revendications 1 à 6, **caractérisé en ce que** la portion de sortie de la lumière de chaque guide de lumière (26) est incorporée dans un élément diffuseur (52) qui est entouré par le réflecteur (54).

8. Appareil selon la revendication 7, **caractérisé en ce que** les réflecteurs (54) sont incorporés en commun dans un diffuseur extérieur (58).

9. Appareil selon l'une des revendications 1 à 6, **caractérisé en ce que** les réflecteurs (54) entourant les portions de sortie de la lumière des guides de lumière (26) sont incorporés dans un diffuseur extérieur (58).

10. Appareil selon l'une des revendications 1 à 9, **caractérisé en ce que** les réflecteurs sont les parois partiellement cylindriques d'alésages qui sont ménagés dans un corps réflecteur (60).

11. Appareil selon l'une des revendications 1 à 10, **caractérisé en ce que** la matière de base pour le diffuseur (52, 58, 62) est choisie dans le groupe constitué par le métacrylate de méthyle, le verre, et une matière plastique souple transparente, et **en ce que** la matière pour les particules de dispersion est choisie dans le groupe constitué par le sulfate de baryum, l'oxyde de magnésium, et l'aluminium.

12. Appareil selon l'une des revendications 1 à 11, **caractérisé en ce que** les particules de diffusion sont fabriquées à partir d'un verre dont l'indice de réfraction est différent de celui de la matière de base ou qui comporte des inclusions de verre.

13. Appareil selon l'une des revendications 1 à 12, **caractérisé en ce que** les guides de lumière (26) se présentent sous la forme de guides de lumière liquides et sont raccordés à l'amenée d'agent de refroidissement.

14. Appareil selon l'une des revendications 1 à 13, **caractérisé en ce que** la sonde est dotée d'un dispositif auxiliaire de positionnement se présentant sous la forme d'au moins un ballon (70).

15. Appareil selon la revendication 14, **caractérisé en ce que** le dispositif auxiliaire de positionnement se trouve au niveau de l'ouverture de sortie de la lumière laser, est transparent pour la lumière laser utilisée, et a une bonne conductibilité thermique.

16. Appareil selon la revendication 14, **caractérisé en ce que** le dispositif auxiliaire de positionnement (70) est agencé à l'extérieur de la zone de rayonnement de la sonde.

17. Appareil selon l'une des revendications 1 à 16, **caractérisé en ce que** la gaine extérieure de la sonde (46) est rigide.

18. Appareil selon l'une des revendications 1 à 16, **caractérisé en ce que** la gaine extérieure de la sonde (46) est souple.

19. Appareil selon l'une des revendications 1 à 18, **caractérisé en ce que** la sonde comporte au moins un capteur de température.

20. Appareil selon l'une des revendications 1 à 19, **caractérisé en ce que** la sonde est fabriquée à partir de matières compatibles du point de vue du spin nucléaire.

21. Appareil selon l'une des revendications 1 à 20, **caractérisé en ce que** la sonde se présente sous la forme d'un cathéter dont l'extrémité distale est dotée d'une pointe appropriée pour percer un tissu fixe.

22. Appareil selon l'une des revendications 1 à 21, **caractérisé en ce que** la sonde comporte au moins une antenne pour émettre et/ou recevoir de l'énergie à haute fréquence.
